# EUROPÄISCHE PATENTANMELDUNG

(11) **EP 2 199 286 A1**
(43) Veröffentlichungstag der Anmeldung: **23.06.2010**
(21) Anmeldenummer: 10157002.6
(22) Anmeldetag: 15.01.2003
(51) Int. Cl.: C07D 235/18

(54) **Verfahren zur Herstellung und Reinigung von 1,7'-dimethyl- 2'-propyl-2,5'-bi-1h-benzimidazol**

(30) Priorität: 18.01.2002 DE 10201725
(62) Teilanmeldung aus: 03729459.2
(71) Anmelder: Boehringer Ingelheim Pharma GmbH & Co. KG, 55216 Ingelheim am Rhein (DE)
(72) Erfinder: BELZER, Werner, 56329, ST. GOAR (DE); DACH, Rolf, 55435, GAU-ALGESHEIM (DE); SCHMIDT, Hartmut, 55595, BOCKENAU (DE); HEITZMANN, Markus, 55218, INGELHEIM AM RHEIN (DE); FACHINGER, Volker, 55569, NUSSBAUM (DE)
(74) Vertreter: Hammann, Heinz

(57) **Zusammenfassung**

Die Erfindung betrifft ein großtechnisch einsetzbares Verfahren zur Herstellung und Reinigung von 1,7'-Dimethyl-2'-propyl-2,5'-bi-1*H*-benzimidazol.

## Beschreibung

Die Erfindung betrifft ein großtechnisch einsetzbares Verfahren zur Herstellung und Reinigung von 1,7'-Dimethyl-2'-propyl-2,5'-bi-1*H*-benzimidazol.

### Hintergrund der Erfindung

Die Herstellung von 1,7'-Dimethyl-2'-propyl-2,5'-bi-1*H*-benzimidazol durch Umsetzung von 2-Propyl-4-methyl-1*H*-benzimidazol-6-carbonsäure mit N-Methyl-o-phenylen-diamin dihydrochlorid ist aus dem J. Med. Chem. (1993), 36(25), 4040-51 und der internationalen Patentanmeldung WO 0063158 bekannt.

1,7'-Dimethyl-2'-propyl-2,5'-bi-1*H*-benzimidazol ist als Zwischenprodukt in der großtechnischen Synthese von Pharmawirkstoffen, insbesondere des Pharmawirkstoffs Telmisartan (Drugs of the future 1997, 22(10), 1112-1116), von großer Bedeutung. Daraus ergibt sich die Anforderung eines hohen Reinheitsgrades. Das aus oben genannter Literatur bekannte Reinigungsverfahren mittels Ethylacetat und Diethylether ist als großtechnisches Verfahren ungeeignet, da beispielsweise Diethylether aufgrund seiner Toxizität, seiner Neigung Peroxide zu bilden und der Explosionsgefahr seiner Dämpfe, z.B. durch elektrostatische Entladung, ein Sicherheitsproblem darstellt, welches eine aufwendige Handhabung erfordert.

Es ist daher die Aufgabe der vorliegenden Erfindung ein großtechnisch einsetzbares Verfahren zur Reinigung für das nach oben beschriebenen Syntheseverfahren hergestellte und den in dieser Patentschrift aufgeführten Varianten 1,7'-Dimethyl-2'-propyi-2,5'-bi-1*H*-benzimidazol bereitzustellen.

### Detaillierte Beschreibung der Erfindung

Überraschenderweise wurde gefunden, dass 1,7'-Dimethyl-2'-propyl-2,5'-bi-1*H-*benzimidazol in hoher Reinheit in einem großtechnisch anwendbaren Verfahren gewonnen werden kann, wenn die wesentlichen Reinigungsschritte unter Verwendung von Kohle durchgeführt werden.

Die Erfindung betrifft daher ein großtechnisch anwendbares Verfahren zur Reinigung von durch Umsetzung von 2-Propyl-4-methyl-1*H*-benzimidazol-6-carbonsäure oder deren Salze mit N-Methyl-o-phenylen-diamin oder dessen Salze hergestelltem 1,7'-Dimethyl-2'-propyl-2,5'-bi-1*H*-benzimidazol, wobei das Rohprodukt mit Kohle behandelt wird.

Erfindungsgemäß bevorzugt ist ein Verfahren, wobei 2-Propyl-4-methyl-1*H-*benzimidazol-6-carbonsäure mit N-Methyl-o-phenylen-diamin oder dessen Salze, vorzugsweise in Form eines Salzes, bevorzugt in Form des Phosphat-, Chlor- oder Bromsalzes, insbesondere bevorzugt in Form des Phosphat- oder Chlorsalzes, besonders bevorzugt in Form des Phosphatsalzes, in Gegenwart von Methansulfonsäure und Phosphorpentoxid umgesetzt wird.

Ferner bevorzugt ist ein Verfahren, wobei die Reaktion bei einer Temperatur von 125 bis 145°C durchgeführt wird.

Besonders bevorzugt ist ein Verfahren, wobei der Reinigungsschritt nach abgeschlossener Reaktion, Hydrolyse und pH-Einstellung durch Zugabe des Reaktionsgemischs zur Kohle erfolgt.

Ferner besonders bevorzugt ist ein Verfahren, wobei der Reinigungsschritt mittels Kohle bei einer Temperatur von 70 bis 80°C durchgeführt wird.

Von besonderer Bedeutung ist ein Verfahren, wobei der Reinigungsschritt bei einem pH-Wert zwischen 0,7 und 1,2 stattfindet.

Ferner von besonderer Bedeutung ist ein Verfahren, wobei die in einem Reinigungsschritt eingesetzte Kohlemenge von 5 bis 20 Gewichtsprozent der eingesetzten 2-Propyl-4-methyl-1*H*-benzimidazol-6-carbonsäure beträgt.

Insbesondere bevorzugt ist ein Verfahren, wobei der Reinigungsschritt nach Filtration oder Zentrifugation des Reaktionsgemisches ein bis dreimal wiederholt wird.

Ferner insbesondere bevorzugt ist ein Verfahren, wobei nach der Reinigung durch Behandlung mit Kohle nacheinander
a) die Zugabe eines organischen Lösungsmittels,
b) die Zugabe einer Base zur Einstellung eines pH-Wertes von 5 bis 6,
c) die Abtrennung der wäßrigen Phase,
d) die Isolierung des 1,7'-Dimethyl-2'-propyl-2,5'-bi-1*H*-benzimidazol aus der organischen Phase durch Kristallisation und anschließende Filtration oder Zentrifugation
erfolgt.

Weiterhin bevorzugt ist ein Verfahren, wobei in Schritt a) als organisches Lösungsmittel Isopropanol eingesetzt wird.

Weiterhin besonders bevorzugt ist ein Verfahren, wobei in Schritt b) die Zugabe einer Base bei einer Temperatur von 70 bis 80°C erfolgt.

Weiterhin insbesondere bevorzugt ist ein Verfahren, wobei in Schritt d) die Kristallisation durch Zugabe von Wasser erfolgt.

1,7'-Dimethyl-2'-propyl-2,5'-bi-1*H*-benzimidazol wird insbesondere zur Herstellung von Telmisartan verwendet. Dabei kann die Herstellung von Telmisartan nach dem in Drugs of the future 1997, 22(10), 1112-1116 beschriebenen Verfahren gemäß folgendem Syntheseschema I erfolgen:

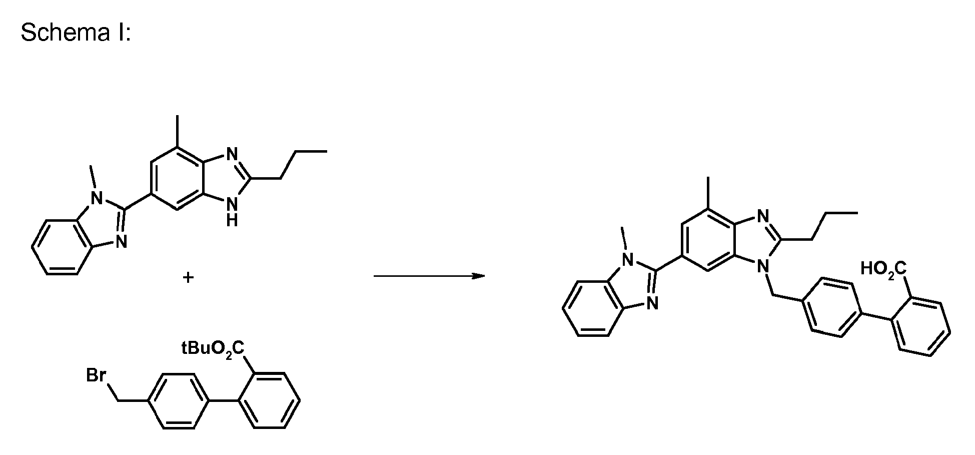

Besonders bevorzugte Ausführungsformen des erfindungsgemäßen Verfahrens stellen die nachfolgenden Varianten A, B, C, D und E dar.

### Variante A:

Zu einer Lösung aus einer organischen Säure, vorzugsweise Methansulfonsäure, Phosphorpentoxid, Thionylchlorid, Sulfonylchlorid, Essigsäureanhydrid oder Polyphosphorsäure, bevorzugt Methansulfonsäure, Phosphorpentoxid oder Thionylchlorid und/oder Mischungen oder Verdünnungen derselben mit inerten organischen Lösungsmitteln, vorzugsweise N-Methylpyrrolidinon, o-, m- oder p-Xylol, besonders bevorzugt N-Methylpyrrolidinon, werden nacheinander unter Rühren bei einer Temperatur von 100 bis 160°C, vorzugsweise bei 115 bis 150°C, insbesondere bei 125 bis 145°C, besonders bevorzugt bei etwa 135°C, 2-Propyl-4-methyl-1*H-*benzimidazol-6-carbonsäure (PMBC) und N-Methyl-o-phenylendiamin (NMPD) oder dessen Salze dosiert. PMBC und NMPD werden in einem molaren Verhältnis von 1,0:0,5 bis 1,0:2,0, vorzugsweise 1,0:0,7 bis 1,0:1,5, insbesondere bevorzugt 1,0:1,0 eingesetzt.

Die Menge an organischer Säure und organischem Lösungsmittel bestimmt sich nach der eingesetzten Menge der PMBC. Pro Mol der PMBC werden bis zu 10 Mol, vorzugsweise 2 bis 8 Mol, besonders bevorzugt etwa 7 Mol an organischer Säure und bis zu 2 Mol, vorzugsweise 0,5 bis 1,5 mol, besonders bevorzugt etwa 1 Mol Phosphorpentoxid eingesetzt.

Nach beendeter Zugabe wird das Gemisch bis zu 10 Stunden, bevorzugt 1 bis 4 Stunden, besonders bevorzugt 3 Stunden bei einer Temperatur von 100 bis 160°C, bevorzugt bei 125 bis 145°C, vorzugsweise 135°C gerührt. Anschließend wird zur Hydrolyse der überschüssigen Säurekomponente (Säureanhydrid, Säurechlorid) bei einer Temperatur von maximal 100°C, bevorzugt max. 90°C Wasser in einem Mengenverhältnis von 1,0:2,0 bis 1,0:0,1, vorzugsweise 1,0:1,4 bis 1,0:0,2 zur eingesetzten organischen Säure, beispielsweise Phosphorpentoxid, zudosiert.

Nach vollständiger Hydrolyse wird durch Zugabe einer Base, vorzugsweise Natriumhydroxid, Natriumcarbonat oder ein Amin, besonders bevorzugt Natriumhydroxid, Natriumcarbonat, Ammoniak, Pyridin oder Triethylamin, besonders bevorzugt Natronlauge oder Ammoniak, insbesondere bevorzugt Natriumhydroxid, ein pH-Wert von 0,2 bis 1,8, bevorzugt von 0,5 bis 1,5, besonders bevorzugt 0,7 bis 1,2, insbesondere bevorzugt 0,9 eingestellt. Die Temperatur des Reaktionsgemisches sollte während der Zugabe 100°C nicht überschreiten, vorzugsweise unter 90°C, besonders bevorzugt bei 70 - 80°C liegen.

Bei konstant bleibendem Temperaturbereich wird das Reaktionsgemisch zur Reinigung mit Kohle, vorzugsweise Aktivkohle, bevorzugt "Aktivkohle trocken", "Aktivkohle feucht" oder "Aktivkohle granuliert", insbesondere bevorzugt "Aktivkohle trocken" der Sorte SX 2, SX Ultra oder L2S, versetzt. Die in dem erfindungsgemäßen Verfahren bevorzugt verwendete Aktivkohle ist beispielsweise erhältlich bei den Firmen Norit, Atofina oder Begerow. Die eingesetzte Kohlemenge pro Reinigungsschritt sollte von 5 bis 20 Gewichtsprozent, vorzugsweise von 6 bis 15 Gewichtsprozent, besonders bevorzugt von 8 bis 12 Gewichtsprozent, insbesondere bevorzugt etwa 10 Gewichtsprozent der eingesetzten Menge der PMBC betragen. Das mit Kohle versetzte Reaktionsgemisch wird mindestens 5 Min, vorzugsweise 5 bis 60 Minuten, bevorzugt 8 bis 50 Minuten besonders bevorzugt 10 bis 30 Minuten bei einer Temperatur von max. 90°C, bevorzugt bei 50 bis 80°C, besonders bevorzugt bei 70 bis 75°C gerührt und anschließend filtriert oder zentrifugiert, vorzugsweise filtriert. Der Reinigungsschritt kann mit dem jeweils filtrierten oder zentrifugierten Reaktionsgemisch ein- bis zehnmal, bevorzugt ein- bis fünfmal, besonders bevorzugt ein- bis dreimal, vorzugsweise zweimal wiederholt werden.

Anschließend erfolgt unter Rühren die Zugabe eines organischen Lösungsmittels, vorzugsweise n-Butanol, tert.-Butanol, 2-Methyl-propanol, n-Propanol, Isopropanol, Ethylacetat, Dichlormethan, Tetrahydrofuran oder Toluol, bevorzugt n-Butanol oder Isopropanol, besonders bevorzugt Isopropanol, zum Reaktionsgemisch. Die Menge des Lösungsmittels wird durch die im Verfahren zuvor eingesetzte Menge an PMBC bestimmt und sollte zwischen 1:1 bis 20:1, bevorzugt 1:1 bis 10:1, besonders bevorzugt 1:1 bis 5:1, vorzugsweise 3:1 betragen.

Nach beendeter Lösungsmittelzugabe wird bei einer Temperatur von max. 100°C, bevorzugt bei 50 bis 90°C, besonders bevorzugt bei 70 bis 80°C, insbesondere bevorzugt bei etwa 75°C, eine Base, vorzugsweise Natriumhydroxid, Natriumcarbonat oder ein Amin, wie z.B. Ammoniak, Pyridin oder Triethylamin, besonders bevorzugt Natriumhydroxid oder Ammoniak, insbesondere bevorzugt Natriumhydroxid, zur Einstellung eines pH-Werts von 4 bis 8, vorzugsweise 5 bis 7, insbesondere bevorzugt etwa 5 bis 6 zudosiert. Der Ansatz wird noch mindestens 5 Minuten, vorzugsweise 10 bis 30 Minuten, bei konstant bleibendem Temperaturbereich gerührt. Nach beendeter Phasentrennung wird die wässrige Phase abgetrennt und verworfen.

Zur Kristallisation des Produkts wird der organischen Phase bei Raumtemperatur oder erhöhter Temperatur, bevorzugt unter Rückfluß, Aceton, Essigester oder Wasser, bevorzugt Aceton oder Wasser, besonders bevorzugt Wasser, zudosiert. Die eingesetzte Wassermenge entspricht 50 bis 200 Volumenprozent, bevorzugt 70 bis 150 Volumenprozent, vorzugsweise Volumenprozent, besonders bevorzugt 80 bis 130 Volumenprozent, insbesondere bevorzugt 120 Volumenprozent des eingesetzten organischen Lösungsmittels.

Nach beendeter Wasserzugabe wird der Ansatz auf eine Temperatur von max. 40, vorzugsweise auf 5 bis 30 °C, besonders bevorzugt auf 10 bis 20°C, insbesondere bevorzugt auf 15°C abgekühlt.

Zur Isolierung des Produkts kann der Ansatz filtriert oder zentrifugiert werden. Das so erhaltene Produkt kann mit einer Waschlösung enthaltend Wasser und ein oder mehrere organische Lösungsmittel ausgewählt aus der Gruppe Aceton, n-Butanol, tert.-Butanol, Cyclohexan, Dichlormethan, Ethylacetat, Isopropanol, Methanol, 2-Methyl-propanol, n-Propanol, Tetrahydrofuran, Toluol oder Xylol, bevorzugt Aceton, Isopropanol oder Ethylacetat, besonders bevorzugt Isopropanol, behandelt werden. In der Regel umfaßt die Waschlösung ein Volumenverhältnis organisches Lösungsmittel/Wasser von 1:0 bis 1:10, vorzugsweise von 1:1 bis 1:8, besonders bevorzugt von 1:1,5 bis 1:6, insbesondere bevorzugt von 1:2. Anschließend wird das Produkt mit Wasser nachgewaschen und bei max. 110°C, bevorzugt bei max. 100°C, besonders bevorzugt bei 75 bis 90°C, bevorzugt im Vakuum, getrocknet.

### Variante B:

In Abwandlung zu Variante A kann die stark saure Reaktionslösung bei etwa einem pH-Wert von 0,2 bis 1,8, bevorzugt von 0,5 bis 1,5, besonders bevorzugt bei 0,7 bis 1,2, insbesondere bevorzugt 0,9, anstatt Kohle zur Reaktionslösung zu geben oder die Reaktionslösung zur Kohle, über mit Kohle gefüllte Fässer oder Kartuschen filtriert werden. Die Reinigung des Filtrats durch Extraktion mit einem organischen Lösungsmittel sowie die weitere Aufarbeitung kann wie in Variante A beschrieben erfolgen.

### Variante C:

In Abwandlung zu Variante A kann, alternativ zur Kohlebehandlung der sauren bis stark sauren Reaktionslösung die organische Produktlösung mit Kohle behandelt werden, beispielsweise Kohle zugeben, zu Kohle zugeben oder über Kohle filtrieren, sowie die weitere Aufarbeitung wie in Variante A beschrieben erfolgen.

### Variante D:

In Abwandlung zu Variante A, B oder C kann die Extraktion mit einer größeren Menge des organischen Lösungsmittels, auch bei niedrigerer Temperatur, durchgeführt und diese vor der Fällung wieder reduziert werden.

### Variante E:

Eine weitere Möglichkeit 1,7'-Dimethyl-2'-propyl-bis-1*H*-benzimidazol als reines Produkt zu erhalten, besteht in der direkten Fällung des Reaktionsprodukts nach abgeschlossener Umsetzung und Hydrolyse durch ausreichende Zugabe einer Base, vorzugsweise Natriumhydroxid, Natriumcarbonat oder ein Amin, wie z.B. Ammoniak, Pyridin oder Triethylamin, besonders bevorzugt Natriumhydroxid oder Ammoniak, insbesondere bevorzugt Natriumhydroxid oder Ammoniak oder Zugabe der Reaktionslösung in eine Lösung der Base. Nach anschließender Filtration wird das Rohprodukt, das getrocknet werden kann, in einer organischen Säure oder einem organischen Lösungsmittel oder einem Gemisch ausgewählt aus der Gruppe Methansulfonsäure, Isopropanol, n-Butanol, Ethylacetat, Methanol oder Toluol, bevorzugt Methansulfonsäure oder Isopropanol, gelöst und die Kohlereinigungschritte mit anschließender weiterer Aufarbeitung wie in Variante A beschrieben durchgeführt. Wird Methansulfonsäure verwendet kann die Fällung auch durch Zugabe einer alkoholischen, beispielsweise methanolischen, Natronlaugelösung oder Einleiten der Produktlösung in eine alkoholische, beispielsweise methanolischen, Natronlaugelösung erfolgen. Bei dieser Variante kann Ascorbinsäure oder BHT (2,6-Di-tert-butyl-4-methoxyphenol) bevorzugt Ascorbinsäure zugesetzt werden.

Die nachfolgenden Beispiele dienen der Illustration der Herstellung und Reinigung von 1,7'-Dimethyl-2'-propyl-2,5'-bi-1H-benzimidazol. Sie stellen lediglich eine mögliche Vorgehensweise dar, ohne die Erfindung auf deren Inhalt zu beschränken.

### Beispiel 1 (Variante A)

### Herstellung und Reinigung von 1,7'-Dimethyl-2'-propyl-2,5'-bi-1H-benzimidazol

70 kg Phosphorpentoxid werden (bei 115 - 145°C) in 300 kg Methansulfonsäure gelöst. Zu dieser Lösung werden bei 125 - 145°C 100 kg 2-Propyl-4-methyl-1H-benzimidazol-6-carbonsäure (PMBC) und 90 kg N-Methyl-o-phenylen-diamin (NMPD) eingetragen. Das Reaktionsgemisch wird bis zu 4 Std. bei dieser Temperatur gerührt und danach auf etwa 80°C abgekühlt. Das Gemisch wird mit etwa 350 I Wasser gequenscht. Durch Zugabe von etwa 180 kg 50%iger Natronlauge wird ein pH-Wert von etwa 1 eingestellt. Die stark saure Reaktionslösung wird mit etwa 10 kg Aktivkohle versetzt und mindestens 5 Min. bei 70 - 80°C nachgerührt. Danach wird die Kohle abfiltriert und mit Wasser gewaschen. Die Kohlung der Lösung wird bis zu 3mal wiederholt. Zum Filtrat werden unter Rühren etwa 400 I Isopropanol gegeben und durch Zugabe von etwa 190 kg 50%iger Natronlauge ein pH-Wert von etwa 5 bis 6 eingestellt. Bei der anschließenden Phasentrennung wird die untere wässrige Phase abgetrennt und verworfen. Zur Fällung des Produkts werden unter Rückfluß zur organischen Phase etwa 420 I Wasser zudosiert. Der Ansatz wird auf etwa 20°C abgekühlt. Das gefällte Produkt wird abzentrifugiert und mit etwa 280 I eines 2:1-Gemisches aus Wasser und Isopropanol und abschließend mit etwa 70 I Wasser gewaschen. Das isolierte Produkt wird mind. 5 Std. bei max. 90°C im Vakuum getrocknet. Es weist eine HPLC-Reinheit von mind. 99,5 Flächenprozent auf. Die Ausbeute beträgt ca. 85% bezogen auf das eingesetzte PMBC.

### Beispiel 2 (Variante E)

### Herstellung und Reinigung von 1,7'-Dimethyl-2'-propyl-2,5'-bi-1H-benzimidazol

70 kg Phosphorpentoxid werden bei 115 - 145°C in 300 kg Methansulfonsäure gelöst. Zu dieser Lösung werden bei 125 - 145°C 100 kg 2-Propyl-4-methyl-1H-benzimidazol-6-carbonsäure (PMBC) und 90 kg N-Methyl-o-phenylen-diamin (NMPD) gegeben. Das Reaktionsgemisch wird bis zu 4 Std. bei dieser Temperatur gerührt und danach auf etwa 80°C abgekühlt. Das Gemisch wird mit etwa 540 I Wasser gequenscht. Zur Fällung des Produktes wird der pH-Wert bei 50 - 60°C mit etwa 370 kg 50%iger Natronlauge auf 5 bis 6 eingestellt. Das gefällte Produkt wird zentrifugiert und mit 780 - 1040 I Wasser gewaschen und bei max. 90°C im Vakuum getrocknet. Die Ausbeute beträgt 80 - 100% bezogen auf das eingesetzte PMBC.

150 kg 1,7'-Dimethyl-2'-propyl-2,5'-bi-1*H*-benzimidazol und 3 kg Ascorbinsäure (optional) werden in 285 I Wasser suspendiert und mit 94 kg Methansulfonsäure versetzt. Zur stark sauren Lösung werden etwa 10 kg Aktivkohle gegeben und die Suspension mindestens 5 Min. bei 50 bis 60°C nachgerührt. Danach wird die Kohle abfiltriert und mit Wasser gewaschen. Die Kohlung der Lösung wird bis zu 6mal wiederholt. Zur Fällung des Produktes wird das Filtrat bei 60 - 80°C unter Rühren in eine Lösung aus etwa 88 kg 45%iger Natronlauge und 150 I Methanol gegeben. Alternativ kann auch die methanolische Natronlauge zum Filtrat gegeben werden. Anschließend wird auf 10 - 25°C abgekühlt und mit Natronlauge ein pH von etwa 9 bis 12 eingestellt. Danach wird das Produkt zentrifugiert und mit etwa 700 I Wasser gewaschen. Das isolierte Produkt wird bei max. 90°C im Vakuum getrocknet. Es weist eine HPLC-Reinheit von ca. 99,0 Flächenprozent auf. Die Ausbeute beträgt ca. 85% bezogen auf das eingesetzte 1,7'-Dimethyl-2'-propyl-2,5'-bi-1*H*-benzimidazol.

### Beispiel 3 (Variante C)

### Reinigung von 1,7'-Dimethyl-2'-propyl-2,5'-bi-1H-benzimidazol

150 kg 1,7'-Dimethyl-2'-propyl-2,5'-bi-1*H*-benzimidazol werden bei 70 - 80°C in 750 I Isopropanol gelöst und mit 7 kg Kohle versetzt. Nach mind. 5 Min. Nachrühren wird die Kohle abgetrennt und mit etwa 70 I Isopropanol und etwa 145 I Wasser gewaschen. Die Kohlung wird bis zu 3mal wiederholt. Zur Fällung des Produktes werden unter Rückfluß zur organischen Phase etwa 420 I Wasser zudosiert. Der Ansatz wird auf etwa 20°C abgekühlt. Das gefällte Produkt wird abzentrifugiert und mit etwa 400 I eines 2:1-Gemisches aus Wasser und Isopropanol und abschließend mit etwa 70 I Wasser gewaschen. Das isolierte Produkt wird mind. 5 Std. bei max. 90°C im Vakuum getrocknet. Es weist eine HPLC-Reinheit von mind. 99,5 Flächenprozent auf. Die Ausbeute beträgt 85 - 95% bezogen auf das eingesetzte 1,7'-Dimethyl-2'-propyl-2,5'-bi-1*H*-benzimidazol.

## Patentansprüche

1. Verfahren zur Reinigung von durch Umsetzung von 2-Propyl-4-methyl-1H-benzimidazol-6-carbonsäure oder deren Salze mit N-Methyl-o-phenylen-diamin oder dessen Salze hergestellten 1,7'-Dimethyl-2'-propyl-2,5'-bi-1*H*-benzimidazol, **dadurch gekennzeichnet, dass** das Rohprodukt mit Kohle behandelt wird.

2. Verfahren nach Anspruch 1 **dadurch gekennzeichnet, dass** 2-Propyl-4-methyl-1H-benzimidazol-6-carbonsäure mit N-Methyl-o-phenylen-diamin oder dessen Salze in Gegenwart von Methansulfonsäure und Phosphorpentoxid umgesetzt wird.

3. Verfahren nach Anspruch 1 oder 2, **dadurch gekennzeichnet, dass** die Reaktion bei einer Temperatur von 125 bis 145°C durchgeführt wird.

4. Verfahren nach einem der Ansprüche 1 bis 3, **dadurch gekennzeichnet, dass** nach abgeschlossener Reaktion, Hydrolyse und pH-Einstellung das Reaktionsgemisch zur Kohle gegeben oder Kohle zum Reaktionsgemisch gegeben wird.

5. Verfahren nach Anspruch 4, **dadurch gekennzeichnet, dass** die Kohlebehandlung bei einer Temperatur von 70 bis 80°C erfolgt.

6. Verfahren nach Anspruch 4 oder 5, **dadurch gekennzeichnet, dass** die Kohlebehandlung bei einem pH-Wert von 0,7 bis 1,2 durchgeführt wird.

7. Verfahren nach einem der Ansprüche 4 bis 6, **dadurch gekennzeichnet, dass** die eingesetzte Kohlemenge von 5 bis 20 Gewichtsprozent des eingesetzten 2-Propyl-4-methyl-1H-benzimidazol-6-carbonsäure beträgt.

8. Verfahren nach einem der Ansprüche 4 bis 7, **dadurch gekennzeichnet, dass** der Reinigungsschritt nach Filtration oder Zentrifugation des Reaktionsgemisches einbis dreimal wiederholt wird.

9. Verfahren nach einem der Ansprüche 4 bis 8, **dadurch gekennzeichnet, dass** nach der Reinigung durch Behandlung mit Kohle nacheinander
a) die Zugabe eines organischen Lösungsmittels,
b) die Zugabe einer Base zur Einstellung eines pH-Wertes zwischen 5 und 6,
c) die Abtrennung der wäßrigen Phase
d) die Isolierung des 1,7'-Dimethyl-2'-propyl-2,5'-bi-1H-benzimidazol aus der organischen Phase durch Kristallisation und anschließende Filtration oder Zentrifugation
erfolgt.

10. Verfahren nach Anspruch 9, **dadurch gekennzeichnet, dass** in Schritt a) als organisches Lösungsmittel Isopropanol eingesetzt wird.

11. Verfahren nach einem der Ansprüche 9-10, **dadurch gekennzeichnet, dass** in Schritt b) die Zugabe einer Base bei einer Temperatur zwischen 70 und 80°C erfolgt.

12. Verfahren nach einem der Ansprüche 9 bis 11, **dadurch gekennzeichnet, dass** in Schritt d) die Kristallisation durch Zugabe von Wasser erfolgt.

13. Verwendung des nach einem Verfahren gemäß einem der Ansprüche 1-12 erhaltenen 1,7'-Dimethyl-2'-propyl-2,5'-bi-1H-benzimidazol zur Herstellung von Telmisartan.
